# EUROPEAN PATENT SPECIFICATION

(11) **EP 4 500 230 B1**
(45) Date of publication and mention of the grant of the patent: **22.07.2026**
(21) Application number: 23715093.3
(22) Date of filing: 27.03.2023
(51) Int. Cl.: G01S 7/52, G01S 15/89, A61B 8/00, B06B 1/02, G10K 11/35

(54) **IMAGING SYSTEM AND METHOD FOR INITIALIZING OPERATION OF AN IMAGING DEVICE**
BILDGEBUNGSSYSTEM UND VERFAHREN ZUR INITIALISIERUNG DES BETRIEBS EINER BILDGEBUNGSVORRICHTUNG
SYSTÈME D'IMAGERIE ET PROCÉDÉ D'INITIALISATION DU FONCTIONNEMENT D'UN DISPOSITIF D'IMAGERIE

(30) Priority: 29.03.2022 US 202263324750 P
(43) Date of publication of application: 05.02.2025
(73) Proprietor: Koninklijke Philips N.V., 5656 AE Eindhoven (NL)
(72) Inventor: HART, Jeffrey, Scott, 5656 AG Eindhoven (NL)
(74) Representative: Philips Intellectual Property & Standards
(86) International application number: PCT/EP2023/057755
(87) International publication number: WO 2023/186759

(56) References cited:
- WO-A1-2013/170053
- JP-A- H10 174 686

## Description

### BACKGROUND

Ultrasound (US) imaging is ubiquitous in a variety of applications, including medical imaging. US imaging devices often comprise an array of piezoelectric transducers that are driven by a motor with various elements such as ball bearings, seals, and gear meshes, to mention only a few.

When an US imaging procedure is begun, and initialization sequence is used to activate the transducers of the US imaging device until steady state operation is reached. This so-called ramp-up is done by gradually increasing the current applied to the motor until steady state operation of the transducer array is realized.

This known initiation sequence can result in a failure to initiate transducers in the imaging device. Specifically, various sources of static friction, such as the ball bearings, seals and gear meshes, result in stiction, and the US imaging device does not begin steady state operation in time resulting in an error signal. Accordingly, the known closed loop servo control of the transducers of the US imaging device cannot overcome the static friction of various components in the US imaging device, after a predetermined error limit is reached , an error code is displayed and actuation of the transducers in the US imaging device is terminated.

The peaking out of the drive current provided to transducer array of known US imaging devices results in inefficiency in the US imaging exam, causing among other things, delay in the initialization of the US imaging device until after multiple attempts to initiate the transducer are realized.

What are needed, therefore, are a device, method and system that overcomes at least the drawbacks of known methods and systems described above.

WO 2013/170053 A1 describes an ultrasound imaging system using a magnetic linear motor driven ultrasound scanner with accurate track and hold operation and/or other motion feedback.

### SUMMARY

According to an aspect of the present disclosure, there is provided an ultrasound imaging system according to claim 1.

According to another aspect of the present disclosure, there is provided a method of initializing an ultrasound device comprising a transducer, wherein the transducer comprises a motor for moving the transducer, according to claim 7.

According to another aspect of the present disclosure, there is provided a tangible, non-transitory computer-readable medium that stores instructions according to claim 12.

### BRIEF DESCRIPTION OF THE DRAWINGS

The representative embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of a system for imaging a portion of a body, according to a representative embodiment.
Fig. 2A is a cross-sectional view of an imaging device according to a representative embodiment.
Fig. 2B is an enlarged view of a portion of the imaging device depicted in Fig. 2A.
Fig. 3A is a simplified block diagram of an imaging device for use in a system for imaging a portion of the body according to a representative embodiment.
Fig. 3B is a simplified block diagram of a controller for use in a system for imaging a portion of the body according to a representative embodiment.
Fig. 4 is a flow-chart of a method for initializing an ultrasound device comprising a transducer in accordance with a representative embodiment.
Fig. 5 is a screen shot showing an initiation sequence of an imaging device of a system for imaging a portion of a body, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

As used in the specification and appended claims, and in addition to their ordinary meanings, the terms 'substantial' or 'substantially' mean to with acceptable limits or degree. For example, 'substantially a square wave signal' means that one skilled in the art would consider the signal to be acceptable for a square wave.

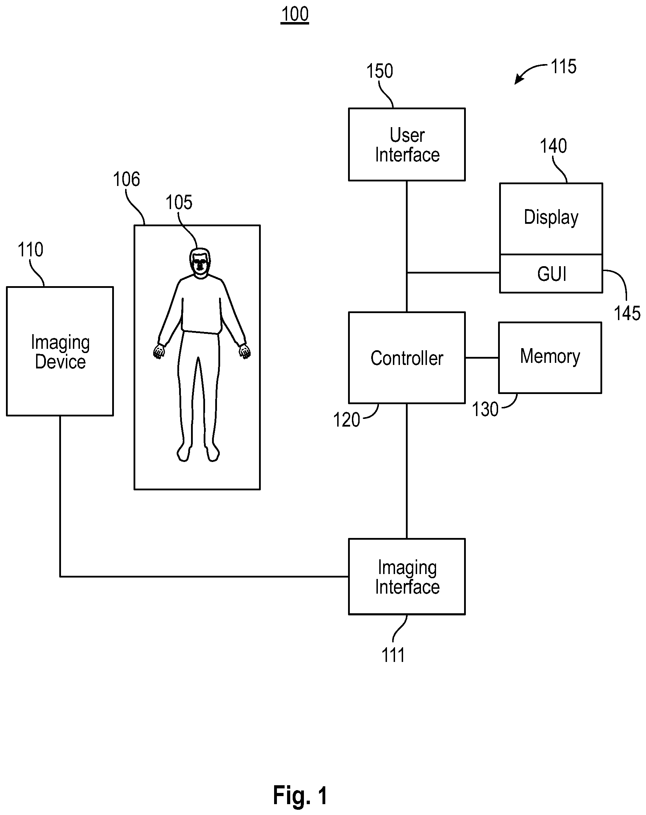

Fig. 1 is a simplified block diagram of an imaging system 100 for imaging a region of interest of a subject, according to a representative embodiment.

Referring to Fig. 1, the imaging system 100 comprises an imaging device 110 and a computer system 115 for controlling imaging of a region of interest in a patient 105 on a table 106. The imaging device 110 is illustratively an ultrasound imaging system capable of providing an ultrasound (US) image scan of a region of interest in the patient 105. Illustratively, the imaging device 110 is of the type commonly used in US imaging procedures. The imaging device 110 is an ultrasound imaging device, and is adapted to provide color Doppler imaging or three dimensional flow volumetry imaging. As described more fully below, the imaging device 110 illustratively comprises a transducer array that may include capacitive micromachined ultrasonic transducers (CMUTs) or piezoelectric transducers formed of materials such as PZT or PVDF, for example. The transducer array may be coupled to a microbeamformer (not shown) in the imaging device, and controls reception of signals by the transducers.

The computer system 115 receives image data from the imaging device 110, and stores and processes the imaging data according to representative embodiments described herein. The computer system 115 comprise a controller 120, a memory 130, a display 140 comprising a graphical user interface (GUI) 145, and a user interface 150. The display 140 may also include a loudspeaker (not shown) to provide audible feedback.

The controller 120 interfaces with the imaging device 110 through an imaging interface 111. The memory 130 stores instructions executable by the controller 120. When executed, and as described more fully below, the instructions cause the controller 120 to allow the user to perform different steps using the GUI 145 or the user interface 150, or both, and, among other tasks, to initialize an ultrasound imaging device comprising a transducer. In addition, the controller 120 may implement additional operations based on executing instructions, such as instructing or otherwise communicating with another element of the computer system 115, including the memory 140 and the display 130, to perform one or more of the above-noted processes.

The controller 120 is representative of one or more processing devices, and is configured to execute software instructions stored in memory 130 to perform functions as described in the various embodiments herein. The controller 120 may be implemented by field programmable gate arrays (FPGAs), application specific integrated circuits (ASICs), a general purpose computer, a central processing unit, a computer processor, a microprocessor, a graphics processing unit (GPU), a microcontroller, a state machine, programmable logic device, or combinations thereof, using any combination of hardware, software, firmware, hard-wired logic circuits, or combinations thereof. Additionally, any processing unit or processor herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems, such as in a cloud-based or other multi-site application. The term computing device should also be interpreted to include a collection or network of computing devices each including a processor or processors. Programs have software instructions performed by one or multiple processors that may be within the same computing device or which may be distributed across multiple computing devices.

The memory 130 may include a main memory and/or a static memory, where such memories may communicate with each other and the controller 120 via one or more buses. The memory 130 stores instructions used to implement some or all aspects of methods and processes described herein. The memory 130 may be implemented by any number, type and combination of random access memory (RAM) and read-only memory (ROM), for example, and may store various types of information, such as software algorithms, which serves as instructions, which when executed by a processor cause the processor to perform various steps and methods according to the present teachings. Furthermore, updates to the methods and processes described herein may also be provided to the computer system 115 and stored in memory 130.

The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

"Memory" is an example of computer-readable storage media, and should be interpreted as possibly being multiple memories or databases. The memory or database may for instance be multiple memories or databases local to the computer, and/or distributed amongst multiple computer systems or computing devices. A computer readable storage medium is defined to be any medium that constitutes patentable subject matter under 35 U.S.C. §101 and excludes any medium that does not constitute patentable subject matter under 35 U.S.C. §101. Examples of such media include non-transitory media such as computer memory devices that store information in a format that is readable by a computer or data processing system. More specific examples of non-transitory media include computer disks and non-volatile memories.

The user interface 150 may include a user and/or network interface for providing information and data output by the controller 120 and/or the memory 130 to the user and/or for receiving information and data input by the user. That is, the user interface 150 enables the user to operate initiate the imaging device as described herein, and to schedule, control or manipulate aspects of the imaging system 100 of the present teachings. Notably, the user interface 150 enables the controller 120 to indicate the effects of the user's control or manipulation. The user interface 150 may include one or more of ports, disk drives, wireless antennas, or other types of receiver circuitry. The user interface 150 may further connect one or more user interfaces, such as a mouse, a keyboard, a mouse, a trackball, a joystick, a microphone, a video camera, a touchpad, a touchscreen, voice or gesture recognition captured by a microphone or video camera, for example.

The display 140 may be a monitor such as a computer monitor, a television, a liquid crystal display (LCD), a light emitting diode (LED) display, a flat panel display, a solid-state display, or a cathode ray tube (CRT) display, or an electronic whiteboard, for example. The display 140 may also provide a graphical user interface (GUI) 145 for displaying and receiving information to and from the user.

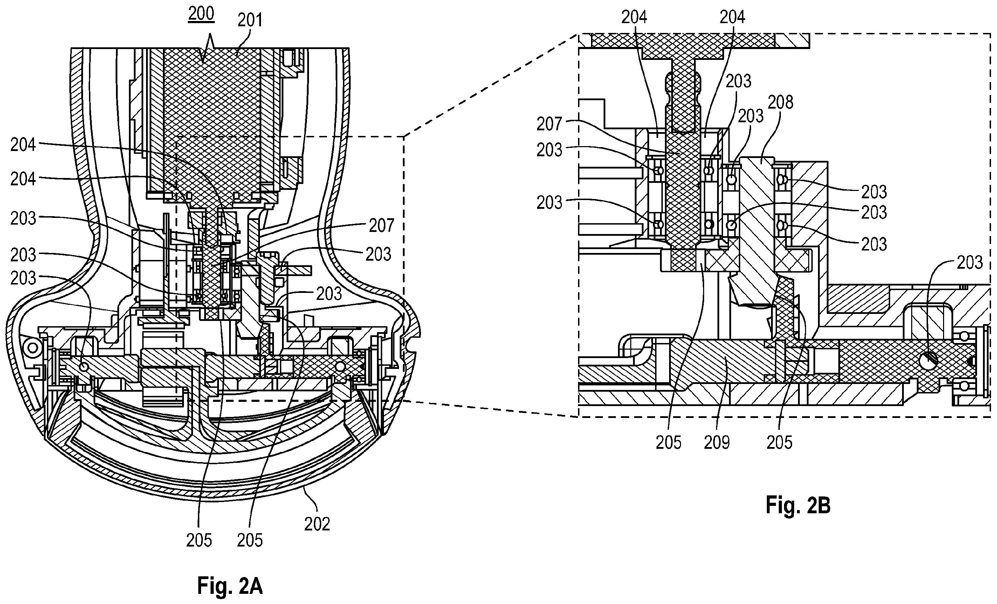

Fig. 2A is a cross-sectional view of a transducer mechanism 200 according to a representative embodiment. Illustratively, the transducer mechanism 200 may be a component of the imaging device 110 of the imaging system 100. Various aspects and details of the transducer mechanism 200 are common to aspects and details of the imaging system 100 described herein, and may not be repeated in order to avoid obscuring the discussion of the present representative embodiment.

The transducer mechanism 200 is illustratively an ultrasound transducer. The transducer mechanism 200 comprises a motor 201, which is driven by a drive amplifier (not shown in Fig. 2A). The drive amplifier provides drive currents that actuate the motor 201 in a transducer array 202. As mentioned above, and as described more fully below, the drive currents provided to the motor 201, and the movement of the transducers of the transducer array 202, provide an initialization drive signal, as well as a steady state drive signal used during an ultrasound examination. Notably, and as described more fully below, in an initialization sequence according to a representative embodiment, the drive amplifier provides a first drive signal to the motor 201, and a second drive signal to the motor 201. Generally, during this illustrative initialization sequence, the first and second drive signals have the same magnitude, but are of opposite sign. As will become clearer as the present description continues, during an initialization sequence according to a representative embodiment, the first and second drive signals have a comparatively short ramp-up time and a comparatively short ramp down time, e.g., less then 1msec. In other contemplated embodiments, the first and second drive signals may have opposite signs but only substantially the same magnitudes, as desired and available in the particular drive amplifier system that implements the invention.

In certain embodiments, and as described more fully below, the first and second drive signals are applied repeatedly until the transducer reaches a dynamic operation, during which various components of the imaging device experience only dynamic friction as discussed more fully below. By way of example, the first and second drive signals may be applied for approximately four to six cycles, which is sufficient to overcome the static friction resistance in an illustrative US system according to the present teachings. The particular number of drive signal cycles may be chosen depending on the nature of the system. Beneficially, and as described more fully below, by the present teachings, initialization of the ultrasound imaging device overcomes stiction of certain mechanical components of the ultrasound imaging device before an error signal is sent from the ultrasound imaging device.

The first and second drive signals provided during the initialization sequence comprise a maximum magnitude, for example corresponding to the maximum magnitude from the voltage supply and the maximum value for the drive amplifier, both of which are described below. By contrast, and as described more fully below, after initialization is complete and dynamic operation is reached, the drive signals provided to the motor 201 of the transducer mechanism 200, are of a lesser magnitude than first and second drive signals. For example, during dynamic operation, the maximum magnitude from the voltage supply is less than 70% of the maximum magnitude from the voltage supply and less than 70% of the maximum value of the drive amplifier. Moreover, during dynamic operation, the output to the motor 201 of the transducer mechanism 200 is further lowered by the function of a proportional, integral, derivation (PID) filter discussed below. Notably, operating the transducer mechanism 200 at a comparably high current in a fixed open loop manner may generate heat, which is deleterious to the components of the transducer mechanism 200 and its function. However, by the present teachings, operation at high current magnitude is comparatively short-lived- and used only to overcome stiction of various components of the transducer mechanism. Once the transducer mechanism 200 overcomes the forces of static friction, and shifts to dynamic operation in closed loop feedback mode, comparatively low current only is required.

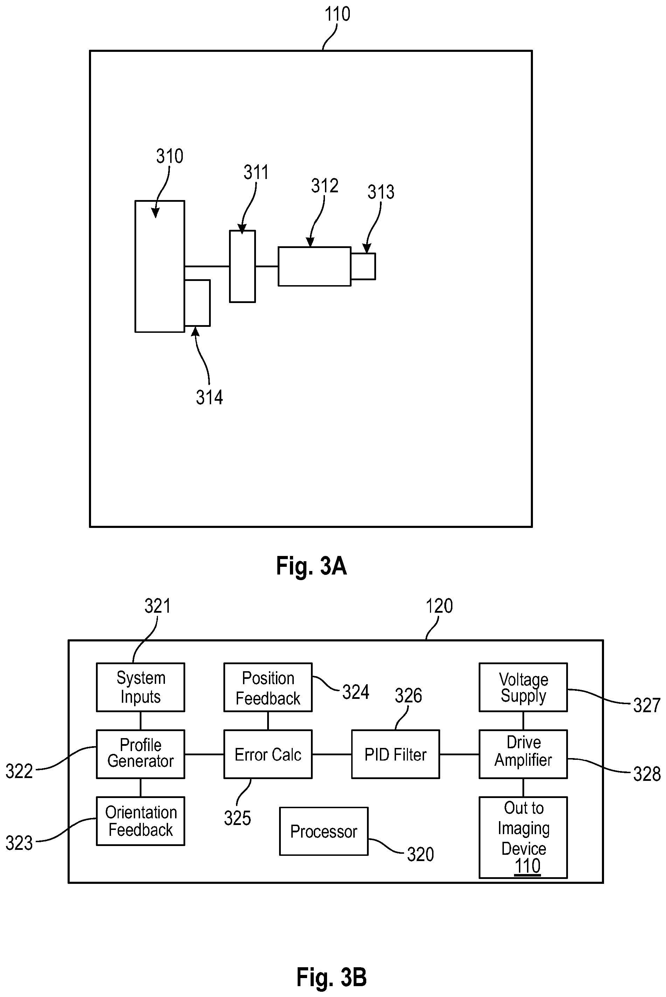

As shown in Fig. 2A, and just by way of example, there are ball bearings 203, a rotary seal 204, and gear meshes 205 in the transducer mechanism 200. While these illustrative elements enable the proper function of the various mechanical elements of the transducer mechanism 200, the ball bearings 203 are sources of dynamic rolling friction, and the rotary seal 204 and gear meshes 205 that are sources of dynamic sliding friction. The sources of dynamic friction are overcome during steady state operation of the transducer mechanism. The ball bearings 203, the rotary seal 204, and the gear meshes 205, and similar elements also may be sources of static friction that can prevent proper initialization of the various moving elements of the transducer mechanism 200. As noted above, these and other sources of static friction must be overcome in order for the various movement elements of the transducer mechanism properly initialize, and without causing an error signal in the closed control loop that effects the function of the transducer mechanism. Notably, and as described more fully in connection with Figs. 3A-3B, closed loop control is applied and is a typical method of controlling the transducer mechanism during dynamic operation.Fig. 2B is an enlarged view of a portion of the transducer mechanism 200 depicted in Fig. 2A to facilitate a better understanding of elements that cause static friction that is overcome in the initialization sequence according to a representative embodiment. The motor 201 of the transducer mechanism 200 is adapted to drive an input shaft 207, which in turn drives various gears and shafts between the input shaft 207, a secondary shaft 208 and a horizontal axle 209, all of which effect the proper function, after initialization, of the transducer array 202.

As noted above, and as described more fully below, at start up, the ball bearings 203, the rotary seal 204 and the gear meshes 205 are sources of static friction that are overcome by the present teachings as described more fully below. It is noted that these sources of static friction are merely illustrative, and the present teachings are contemplated for use to overcome more and fewer sources of static friction in transducer mechanism to which the present teachings are applied.

Fig. 3A is a simplified block diagram of the imaging device 110 for use in the imaging system 100 for imaging a portion of the body according to a representative embodiment. Various aspects and details of the imaging device 110 described presently are common to aspects and details of the imaging system 100 and transducer mechanism 200 described herein, and may not be repeated in order to avoid obscuring the discussion of the present representative embodiment.

The imaging device 110 comprises a sensor 310, which is illustratively a transducer sensor such as described above. The sensor 310 is connected to a transducer mechanism 311, and may be based on the transducer mechanism 200 described above. The transducer mechanism 311 is connected to a motor 312 and may be based on the motor 201 described above. Notably, however, the sensor 310, the transducer mechanism 311, and the motor 312 may comprise other components than those described above. Moreover, the sensor 310, the transducer mechanism 311, and the motor 312 may be based on other technologies known to those of ordinary skill in the art. As will be appreciated, common to all imaging devices to which the present teachings are applied, elements that are the sources of stiction during initialization/start up are present and may interfere with the proper function of the imaging device in the imaging system. These sources of static friction are overcome by temporarily disabling the closed loop control of the imaging device 110, and applying the principles of the present teachings until dynamic operation is achieved. (As alluded to above, dynamic operation are used herein means only dynamic friction of various components of the transducer mechanism remains.)

The imaging device 110 also comprises a position feedback sensor 313 connected to the motor 312, and an orientation feedback sensor 314 connected to the sensor 310.

The position feedback sensor 313 provides output signals used by the controller 120 of the imaging system 100 to calculate the position, velocity and acceleration of the sensor, and to provide feedback of these measures at initialization and operation of the sensor.

The orientation feedback sensor 314 is illustratively connected to the horizontal axle 209 and comprises a magnet and a Hall Effect sensor that is mounted on a stationary side of the imaging device. As is known, the orientation feedback sensor 314 defines the center plane or home position of the sensor 310. As such, the orientation feedback sensor 314 defines where a top dead center position is on the sensor 310, providing an origin for positive or negative movement of the sensor relative to that position. Stated somewhat differently, the orientation feedback sensor 314 provides an origin or datum for a coordinate axis against which the position, velocity and acceleration of the sensor 310 may be measured.

Fig. 3B is a simplified block diagram of the controller 120 for use in a system for imaging a portion of the body according to a representative embodiment. Various aspects and details of the controller 120 described presently are common to aspects and details of the imaging system 100, transducer mechanism 200, and the imaging device 110 described herein. These common aspects and details may not be repeated in order to avoid obscuring the discussion of the present representative embodiment.

The controller 120 comprises a processor 320, which accesses computer-executable instructions from the memory 130 and executes the instructions to provide the various computations in "blocks" described in connection with Fig. 3B. These "blocks" may be implemented in hardware, or may be elements of a tangible non-transitory computer readable medium (e.g., memory 130) and computations effected by the processor 320 when executing instructions stored in memory 130, or both. The processor 320 may comprise a processor described above in connection with Fig. 1, and in addition to providing the various computations of Fig. 3B, upon execution of the instructions stored in the memory 130, carries out the initialization sequence of a method for initializing the imaging device 110 described below.

The controller 120 comprises system inputs block 321, which are provided to the profile generator block 322. The system inputs block 321 are provided by the user and may be received from memory 130. The system inputs block 321 comprises certain common inputs to many ultrasound imaging systems and may include a mode of operation, position ranges, velocities, accelerations, of the components of the transducer mechanism 200. The system inputs block 321 may also comprise a set of input parameters provided by the user.

The orientation feedback block 323 comprises data from the orientation feedback sensor 314. These data may also be stored in memory 130, and provide position information of the sensor 310 relative to the origin in the coordinate system from the of the coordinate system of the sensor 310 (i.e., positive or negative side of the orientation in the coordinate system).

The system inputs block 321 and the orientation feedback block 323 are provided to the profile generator block 322, and the processor 320 computes an ideal state for the imaging device 110 for dynamic operation. To this end, and just by way of illustration, operation the user may input a movement frequency of 2 Hz movement over a range of 45 degrees. The processor 320 executes instructions stored in memory 130 to calculate the ideal state to be moving across that range of motion at that speed.

The ideal state from the profile generator block 322 is input to the error calculation block 325. The position feedback data block 324 stores the data from the position feedback sensor 313, and is provided to the error calculation block 325. The processor 320 then calculates the error at error calculation block 325. The processor 320 calculates the error by comparing the ideal state with the position feedback data and determines the error at the error calculation block 325.

An output calculation from the error calculation block 325 is provided to a proportional, integral derivative (PID) filter block 326. Here the processor 320 improves the control loop by minimizing the error and enables tuning the imaging system 100 using known methods.

The output from the PID filter block 326 is provided to a drive amplifier 328, which is connected to a voltage supply 327. Voltage supply 327 may supply alternating current or direct current power sufficient for transducer drive operation to the drive amplifier 328. Signals from the drive amplifier 328 are output to the imaging device 110. As described more fully below, the signals from the drive amplifier 328 are used to initialize the imaging device by overcoming the various sources of static friction, and are adjusted after normal operation is reached to carry out the imaging by the imaging system 100.

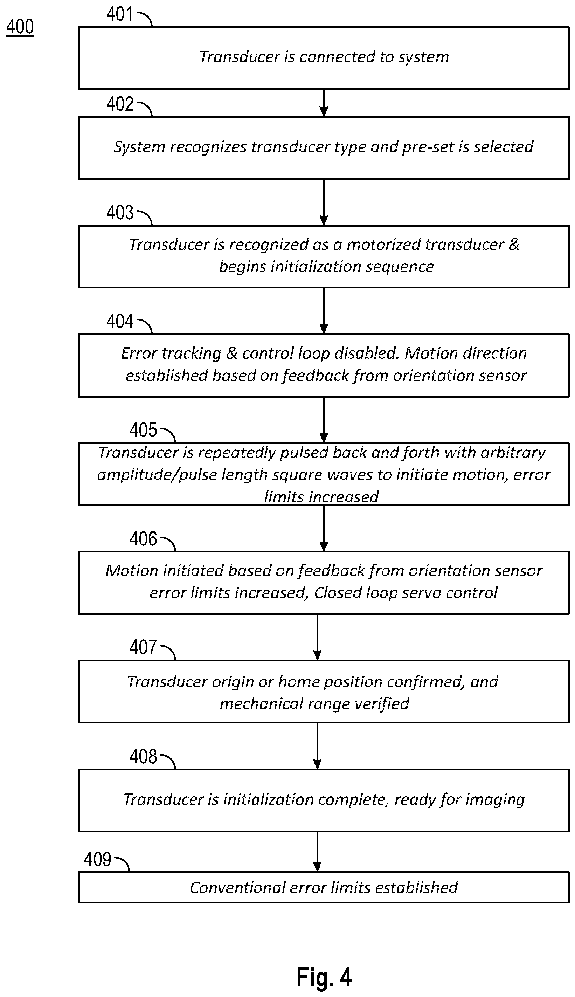

Fig. 4 is a flow-chart of a method 400 for initializing an ultrasound device comprising a transducer in accordance with a representative embodiment. Various aspects and details of the method 400 are common to aspects and details of the imaging system 100, transducer mechanism 200, the imaging device 110 and the controller 120 described herein. These common aspects and details may not be repeated in order to avoid obscuring the discussion of the present representative embodiment. Furthermore, various aspects of the method are carried out by the controller 120 and the processor 320 thereof. Most notably, the various steps including recognition, initialization, and operation described in connection with the method 400 are stored as instructions that are executable by the controller 120/processor 320 are stored in memory 130, and when executed by the controller 120/processor 320 cause the controller/processor to carry out various aspects of the method 400.

At 401, the method 400 begins with connection of the imaging device 110 comprising transducer mechanism 200 to the imaging system 100.

At 402, the method continues with the imaging system 100 recognizing the type of transducer of the imaging device 110, and model specific operational parameters (i.e., input parameters) are selected and stored into memory 130.

At 403, the method continues with recognizing the imaging device 110 is a motorized ultrasound transducer such as discussed more fully above, and an initial portion of an initialization sequence according to a representative embodiment is begun. At this initial portion of the initialization sequence, the imaging device 110 undergoes a homing sequence to make sure the sensor 310 is at the origin of its coordinate system (i.e., top dead center) to start.

Notably, in known systems, as noted above, the initialization can fail because the static friction of various components is not overcome by the motor of the motorized ultrasound transducer and an error code is sent to the controller. When this occurs, as noted above, in known systems, the initialization step is repeated in the hope that the stiction of the various components of the motorized ultrasound transducer are released. By the present teachings, this failure is avoided by application of a distinct initialization sequence that begins at 404 with the disabling of the prior art error tracking and closed control loop. By contrast to known imaging devices, the control loop is open during which no error calculations are performed in the error calculation block 325, and no PID filtering is performed by the PID filter block 326. As such, when the control loop is open, the input signal to the drive amplifier 328 used during dynamic operation is bypassed. Instead, drive amplifier 328 applies pairs of first and second drive signals until initialization is complete. Illustratively, connections to the PID filter block 326 and the attendant PID error correction loop are disabled to avoid an error signal causing the imaging device 110 to shut off.

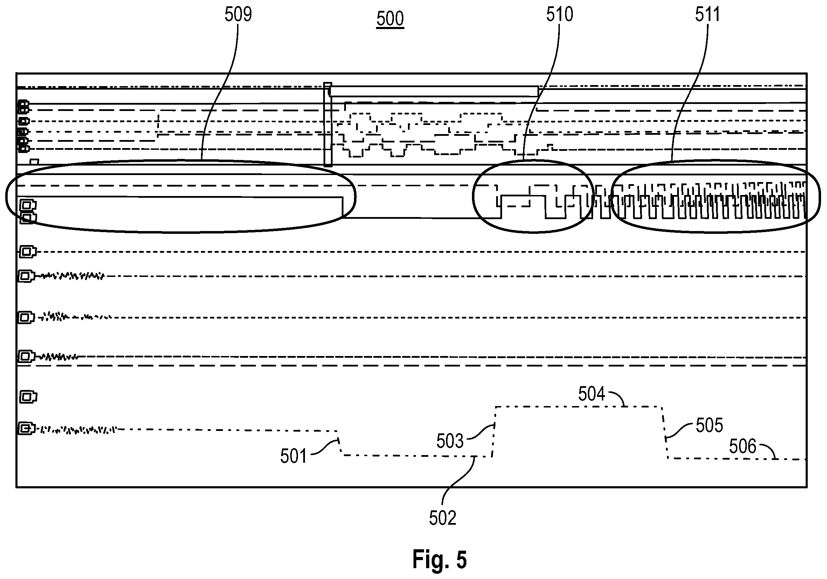

At 405, the method continues with repeatedly driving the imaging device 110 alternating with a comparatively high positive current/voltage from the drive amplifier 328 followed alternatingly with a comparative high negative current/voltage. As noted above, and as shown and described in connection with Fig. 5, in a representative embodiment, the drive signal applied to the imaging device 110 is a square wave signal where the magnitudes of the applied current/voltages are equal in magnitude, but opposite in sign. The duration of each pulse of the applied square wave signal (or substantially square wave signal) can be varied based on inputs to the controller 120 by the user. In accordance with a representative embodiment, each maximum pulse (impulse) is applied for approximately 5 msec to approximately 25 msec and the magnitude of the applied pulse (i.e., the magnitude of the first or second signal) is the maximum magnitude from the voltage supply 327 and the maximum value from the drive amplifier 328. As indicated above, the number of optimal number of applied pulses may vary depending on the type of transducer mechanism to be operated.

After initialization at 405 is complete, and/or when the static friction of the various components of the transducer mechanism 200 are overcome, the method continues at 406 based on feedback from the orientation feedback sensor 314. Notably, the output level from the orientation feedback sensor is used to determine direction of travel ensuring movement toward the centerline or origin of transducer.

At 407, the method continues with the resetting of the imaging device 110 to the origin of the coordinate system of the sensor 310. As such, the origin of the coordinate system of the sensor 310 is confirmed. Next, the mechanical range of the imaging device is verified to confirm functionality of positioning system.

At 408, the initialization sequence of the imaging device 110 is complete, and ready for an imaging procedure with establishment of error limits during dynamic operation at 409. At this step 408, controller 120 shifts the above described open loop initialization sequence to the closed loop transducer control for steady state operation.

Fig. 5 is a screen shot 500 from a diagnostic measuring device (e.g., an oscilloscope) showing the various electronic voltage and current signals applied during an initiation sequence of an imaging device of a system for imaging a portion of a body, according to a representative embodiment. Various aspects and details of the method 400 are common to aspects and details of the imaging system 100, transducer mechanism 200, the imaging device 110, the controller 120 and the method 400 described herein.

The screen shot 500 depicts an initialization sequence beginning with drive signal 501 depicting the applied current/voltage to the imaging device. This drive signal 501 that may be provided by drive amplifier 328 initially ramps down and turns negative and has a magnitude as described more fully above. At 502, the negative signal is shown and has a comparatively steady value. At 503, the drive signal 501 abruptly changes sign and ramps up to a peak positive magnitude at 504. Notably, and as described more fully above, the drive current/voltage of the drive signal 501 is equal in magnitude, but opposite in direction, to the peak positive magnitude.

As will be appreciated from a review of Fig. 5, the drive signal 501 begins the application of a substantially square wave signal with maximum negative magnitude at 502 and maximum positive magnitude at 504 with ramp up and ramp down of the applied signal. By "substantially square wave," it is noted that the ramp ups and ramp downs, while ideally are vertical, may not be perfectly vertical (i.e., may not be perfectly infinite in slope); and maximum negative magnitude at 502 and maximum positive magnitude at 504, while ideally are flat, may not be perfectly flat (i.e., may not have a slope of 0). Moreover, and as alluded to above, maximum negative magnitude signal at 502 and maximum positive magnitude signal at 504 may not be perfectly equal in magnitude, and as such may be substantially the same magnitude (e.g., within ± 10% of each other). Furthermore, while the duration of the maximum negative magnitude signal at 502 and maximum positive magnitude signal at 504 are illustratively identical, their durations are not necessarily always equal. Their durations can be substantially the same (e.g., within ± 10% of each other), or could be significantly different in duration.

At 505, the drive signal again ramps down to a second negative peak at 506.

The process of ramping up and ramping down the drive signal 501 is continued until the stiction of the various components of the transducer mechanism 200 of the imaging device 110 is overcome. The determination of when the stiction is overcome may be inferred from a set number of drive signal cycles, or can alternatively be shown from a review of the feedback signals 509, 510, 511 from the imaging device. Notably, feedback signal 509 shows a region where the transducer mechanism is static with no drive signal applied. By contrast, the oscillations of the position feedback sensor 313 in feedback signals 510 are comparatively long in duration. This is indicative low velocity and of stiction of the various components of the transducer mechanism 200 of the imaging device 110 during initialization. Feedback signal 511 shows oscillations of the sensor are comparatively short in duration. This is indicative of achieving higher velocity operation, transition to dynamic friction and dynamic operation, and readiness for the ultrasound procedure to begin.

As will be appreciated by one of ordinary skill in the art having the benefit of the present disclosure, the systems and methods of the present teachings provide an improvement in the initialization of imaging devices that include certain components that cause stiction of the imaging devices during initialization. For example, compared to known methods and systems, various aspects of a protocol including the beginning, duration and termination of a step in the protocol can be facilitated during the generation of the protocol, or during implementation of the protocol, or both. Moreover, errors that can result from human interaction with an imaging system can be reduced thereby reducing the need to repeat procedures, and reducing the time required to complete an imaging procedure. Notably, these benefits are illustrative, and other advancements in the field of medical imaging will become apparent to one of ordinary skill in the art having the benefit of the present disclosure.

## Claims

1. An ultrasound imaging system (100), comprising:
an ultrasound imaging device (110) comprising a transducer, wherein the transducer comprises a motor (201) for moving the transducer;
a controller (120) comprising a processor (320), the controller (120) being connected to the ultrasound imaging device (110), and adapted to drive the transducer of the ultrasound imaging device (110); and
a memory (130) that stores instructions, which when executed by the processor (320), cause the controller (120) to:
initialize the ultrasound imaging device (110) by applying to the motor (201) of the transducer a first drive signal (501) having a magnitude and a sign followed by a second drive signal (502) having substantially the magnitude of the first drive signal (501) ;
**characterized in that**
the second drive signal (502) has an opposite sign to
the sign of the first drive signal (501), the first drive signal (501) and the second drive signal (502) provide a substantially square wave, and the magnitudes of the first and second drive signals (501, 502) comprise a maximum magnitude.

2. The ultrasound imaging system (100) as claimed in claim 1, wherein the first drive signal (501) and the second drive signal (502) have substantially the same duration.

3. The ultrasound imaging system (100) as claimed in claim 1, wherein the instructions further cause the controller (120) to repeatedly apply the first and second drive signals (501, 502) until the transducer reaches dynamic operation.

4. The ultrasound imaging system (100) as claimed in claim 3, wherein when the transducer reaches dynamic operation, the instructions cause the controller (120) to operate in a closed loop mode of operation.

5. The ultrasound imaging system (100) according to claim 1, wherein initialization of the ultrasound imaging device (110) overcomes stiction of mechanical components of the ultrasound imaging device (110).

6. The ultrasound imaging system (100) according to claim 1, further comprising a voltage supply and a drive amplifier.

7. A method (400) of initializing an ultrasound imaging device (110) comprising a transducer, wherein the transducer comprises a motor for moving the transducer, the method (400) comprising:
applying to the motor of the transducer, with a controller (120) comprising a processor (320), a first drive signal (501) having a magnitude and a sign followed by a second drive signal (502) having substantially the magnitude of the first drive signal (501);
**characterized in that**
the second drive signal (502) has an opposite sign to the sign of the first drive signal (501), the first drive signal (501) and the second drive signal (502) provide a substantially square wave,
and the magnitudes of the first and second drive signals (501, 502) comprise a maximum magnitude.

8. The method (400) as claimed in claim 7, wherein the first drive signal (501) and the second drive signal (502) have substantially the same duration.

9. The method (400) as claimed in claim 7, further comprising, with the controller (120), repeatedly applying the first and second drive signals (501, 502) until the transducer reaches dynamic operation.

10. The method (400) as claimed in claim 9, wherein after the transducer reaches dynamic operation, the method comprises operating in a closed loop mode of operation.

11. The method (400) as claimed in claim 7, wherein initializing of the ultrasound imaging device (110) overcomes stiction of mechanical components of the ultrasound imaging device (110).

12. A tangible, non-transitory computer-readable medium that stores instructions, which when executed by the processor (320) of the controller (120) of the ultrasound imaging system (100) according to claim 1, cause the controller (120) to:
initialize the ultrasound imaging device (110) by applying to the motor (201) of the transducer a first drive signal (501) having a magnitude and a sign followed by a second drive signal (502) having substantially the magnitude of the first drive signal (501);
**characterized in that**
the second drive signal (502) has an opposite sign to the sign of the first drive signal (501), the first drive signal (501) and the second drive signal (502) provide a square wave, and the magnitudes of the first and second drive signals (501, 502) comprise a maximum magnitude.

13. The tangible, non-transitory computer-readable medium as claimed in claim 12, wherein the instructions further cause the controller (120) to repeatedly apply the first and second drive signals (501, 502) until a transducer reaches dynamic operation.

14. The tangible, non-transitory computer-readable medium as claimed in claim 13, wherein when the transducer reaches dynamic operation, the instructions cause the controller (120) to operate in a closed loop mode of operation.

15. The tangible, non-transitory computer-readable medium according to claim 12, wherein initialization of the ultrasound imaging device (110) overcomes stiction of mechanical components of the ultrasound imaging device (110).

## Patentansprüche

1. Ultraschallbildgebungssystem (100), umfassend:
eine Ultraschallbildgebungsvorrichtung (110), die einen Wandler umfasst, wobei der Wandler einen Motor (201) zum Bewegen des Wandlers umfasst;
eine Steuereinheit (120), die einen Prozessor (320) umfasst, wobei die Steuereinheit (120) mit der Ultraschallbildgebungsvorrichtung (110) verbunden und zur Ansteuerung des Wandlers der Ultraschallbildgebungsvorrichtung (110) ausgelegt ist; und
einen Speicher (130), der Anweisungen speichert, die, wenn sie vom Prozessor (320) ausgeführt werden, die Steuereinheit (120) veranlassen zum:
Initialisieren der Ultraschallbildgebungsvorrichtung (110), indem an den Motor (201) des Wandlers ein erstes Ansteuersignal (501) mit einer bestimmten Amplitude und einem bestimmten Vorzeichen, gefolgt von einem zweiten Ansteuersignal (502) mit im Wesentlichen der Amplitude des ersten Ansteuersignals (501) angelegt wird,
**dadurch gekennzeichnet, dass**
das zweite Ansteuersignal (502) ein entgegengesetztes Vorzeichen zum Vorzeichen des ersten Ansteuersignals (501) aufweist, das erste Ansteuersignal (501) und das zweite Ansteuersignal (502) im Wesentlichen eine Rechteckwelle bereitstellen, und die Amplituden des ersten und des zweiten Ansteuersignals (501, 502) eine maximale Amplitude umfassen.

2. Ultraschallbildgebungssystem (100) nach Anspruch 1, wobei das erste Ansteuersignal (501) und das zweite Ansteuersignal (502) im Wesentlichen die gleiche Dauer aufweisen.

3. Ultraschallbildgebungssystem (100) nach Anspruch 1, wobei die Anweisungen weiter die Steuereinheit (120) veranlassen, das erste und das zweite Ansteuersignal (501, 502) wiederholt anzulegen, bis der Wandler den dynamischen Betrieb erreicht.

4. Ultraschallbildgebungssystem (100) nach Anspruch 3, wobei, wenn der Wandler den dynamischen Betrieb erreicht, die Anweisungen die Steuereinheit (120) veranlassen, in einem geschlossenen Regelkreis zu arbeiten.

5. Ultraschallbildgebungssystem (100) nach Anspruch 1, wobei die Initialisierung der Ultraschallbildgebungsvorrichtung (110) die Haftreibung mechanischer Komponenten der Ultraschallbildgebungsvorrichtung (110) überwindet.

6. Ultraschallbildgebungssystem (100) nach Anspruch 1, weiter umfassend eine Spannungsversorgung und einen Ansteuerungsverstärker.

7. Verfahren (400) zum Initialisieren einer Ultraschallbildgebungsvorrichtung (110), die einen Wandler umfasst, wobei der Wandler einen Motor zum Bewegen des Wandlers umfasst, wobei das Verfahren (400) Folgendes umfasst:
Anlegen, an den Motor des Wandlers mit einer Steuereinheit (120), die einen Prozessor (320) umfasst, eines ersten Ansteuersignals (501) mit einer Amplitude und einem Vorzeichen, gefolgt von einem zweiten Ansteuersignal (502) mit im Wesentlichen der Amplitude des ersten Ansteuersignals (501);
**dadurch gekennzeichnet, dass**
das zweite Ansteuersignal (502) ein entgegengesetztes Vorzeichen zum Vorzeichen des ersten Ansteuersignals (501) aufweist, das erste Ansteuersignal (501) und das zweite Ansteuersignal (502) im Wesentlichen eine Rechteckwelle bereitstellen, und die Amplituden des ersten und des zweiten Ansteuersignals (501, 502) eine maximale Amplitude umfassen.

8. Verfahren (400) nach Anspruch 7, wobei das erste Ansteuersignal (501) und das zweite Ansteuersignal (502) im Wesentlichen die gleiche Dauer aufweisen.

9. Verfahren (400) nach Anspruch 7, weiter umfassend, mit der Steuereinheit (120), das wiederholte Anlegen des ersten und zweiten Ansteuersignals (501, 502), bis der Wandler den dynamischen Betrieb erreicht.

10. Verfahren (400) nach Anspruch 9, wobei das Verfahren, nachdem der Wandler den dynamischen Betrieb erreicht hat, den Betrieb in einem geschlossenen Regelkreis umfasst.

11. Verfahren (400) nach Anspruch 7, wobei die Initialisierung der Ultraschallbildgebungsvorrichtung (110) die Haftreibung mechanischer Komponenten der Ultraschallbildgebungsvorrichtung (110) überwindet.

12. Greifbares, nichtflüchtiges, computerlesbares Medium, das Anweisungen speichert, die, wenn sie vom Prozessor (320) der Steuereinheit (120) des Ultraschallbildgebungssystems (100) nach Anspruch 1 ausgeführt werden, die Steuereinheit (120) veranlassen zum:
Initialisieren der Ultraschallbildgebungsvorrichtung (110), indem an den Motor (201) des Wandlers ein erstes Ansteuersignal (501) mit einer bestimmten Amplitude und einem bestimmten Vorzeichen, gefolgt von einem zweiten Ansteuersignal (502) mit im Wesentlichen der Amplitude des ersten Ansteuersignals (501) angelegt wird;
**dadurch gekennzeichnet, dass**
das zweite Ansteuersignal (502) ein entgegengesetztes Vorzeichen zum Vorzeichen des ersten Ansteuersignals (501) aufweist, das erste Ansteuersignal (501) und das zweite Ansteuersignal (502) eine Rechteckwelle bereitstellen, und die Amplituden des ersten und des zweiten Ansteuersignals (501, 502) eine maximale Amplitude umfassen.

13. Greifbares, nichtflüchtiges, computerlesbares Medium nach Anspruch 12, wobei die Anweisungen weiter die Steuereinheit (120) veranlassen, das erste und das zweite Ansteuersignal (501, 502) wiederholt anzuwenden, bis ein Wandler den dynamischen Betrieb erreicht.

14. Greifbares, nichtflüchtiges, computerlesbares Medium nach Anspruch 13, wobei die Anweisungen, wenn der Wandler den dynamischen Betrieb erreicht, die Steuereinheit (120) veranlassen, in einem geschlossenen Regelkreis zu arbeiten.

15. Greifbares, nichtflüchtiges, computerlesbares Medium nach Anspruch 12, wobei die Initialisierung der Ultraschallbildgebungsvorrichtung (110) die Haftreibung mechanischer Komponenten der Ultraschallbildgebungsvorrichtung (110) überwindet.

## Revendications

1. Système d'imagerie ultrasonore, comprenant :
un dispositif d'imagerie ultrasonore (110) comprenant un transducteur, dans lequel le transducteur comprend un moteur (201) pour déplacer le transducteur ;
un dispositif de commande (120) comprenant un processeur (320), le dispositif de commande (120) étant connecté au dispositif d'imagerie ultrasonore (110) et adapté pour entraîner le transducteur du dispositif d'imagerie ultrasonore (110) ; et
une mémoire (130) qui stocke des instructions lesquelles, lorsqu'elles sont exécutées par le processeur (320), amènent le dispositif de commande (120) à :
initialiser le dispositif d'imagerie ultrasonore (110) en appliquant au moteur (201) du transducteur un premier signal d'entraînement (501) présentant une amplitude et un signe suivi d'un deuxième signal d'entraînement (502) présentant sensiblement l'amplitude du premier signal d'entraînement (501) ;
**caractérisé en ce que**
le deuxième signal d'entraînement (502) présente un signe opposé au signe du premier signal d'entraînement (501), le premier signal d'entraînement (501) et le deuxième signal d'entraînement (502) fournissent une onde sensiblement carrée, et les amplitudes des premier et deuxième signaux d'entraînement (501, 502) comprennent une amplitude maximale.

2. Système d'imagerie ultrasonore (100) selon la revendication 1, dans lequel le premier signal d'entraînement (501) et le deuxième signal d'entraînement (502) présentent sensiblement la même durée.

3. Système d'imagerie ultrasonore (100) selon la revendication 1, dans lequel les instructions amènent en outre le dispositif de commande (120) à appliquer de manière répétée les premier et deuxième signaux d'entraînement (501, 502) jusqu'à ce que le transducteur atteigne un fonctionnement dynamique.

4. Système d'imagerie ultrasonore (100) selon la revendication 3, dans lequel lorsque le transducteur atteint un fonctionnement dynamique, les instructions amènent le dispositif de commande (120) à fonctionner dans un mode de fonctionnement en boucle fermée.

5. Système d'imagerie ultrasonore (100) selon la revendication 1, dans lequel l'initialisation du dispositif d'imagerie ultrasonore (110) surmonte la friction de composants mécaniques du dispositif d'imagerie ultrasonore (110).

6. Système d'imagerie ultrasonore (100) selon la revendication 1, comprenant en outre une alimentation en tension et un amplificateur d'entraînement.

7. Procédé (400) d'initialisation d'un dispositif d'imagerie ultrasonore (110) comprenant un transducteur, dans lequel le transducteur comprend un moteur pour déplacer le transducteur, le procédé (400) comprenant :
l'application au moteur du transducteur, à l'aide d'un dispositif de commande (120) comprenant un processeur (320), d'un premier signal d'entraînement (501) présentant une amplitude et un signe suivi d'un deuxième signal d'entraînement (502) présentant sensiblement l'amplitude du premier signal d'entraînement (501) ;
**caractérisé en ce que**
le deuxième signal d'entraînement (502) présente un signe opposé au signe du premier signal d'entraînement (501), le premier signal d'entraînement (501) et le deuxième signal d'entraînement (502) fournissent une onde sensiblement carrée, et les amplitudes des premier et deuxième signaux d'entraînement (501, 502) comprennent une amplitude maximale.

8. Procédé (400) selon la revendication 7, dans lequel le premier signal d'entraînement (501) et le deuxième signal d'entraînement (502) présentent sensiblement la même durée.

9. Procédé (400) selon la revendication 7, comprenant en outre, à l'aide du dispositif de commande (120), une application répétée des premier et deuxième signaux d'entraînement (501, 502) jusqu'à ce que le transducteur atteigne un fonctionnement dynamique.

10. Procédé (400) selon la revendication 9, dans lequel après que le transducteur a atteint un fonctionnement dynamique, le procédé comprend un fonctionnement dans un mode de fonctionnement en boucle fermée.

11. Procédé (400) selon la revendication 7, dans lequel l'initialisation du dispositif d'imagerie ultrasonore (110) surmonte la friction de composants mécaniques du dispositif d'imagerie ultrasonore (110).

12. Support tangible, non transitoire et lisible par ordinateur, qui stocke des instructions qui, lorsqu'elles sont exécutées par le processeur (320) du dispositif de commande (120) du système d'imagerie ultrasonore (100) selon la revendication 1, amènent le dispositif de commande (120) à :
initialiser le dispositif d'imagerie ultrasonore (110) en appliquant au moteur (201) du transducteur un premier signal d'entraînement (501) présentant une amplitude et un signe suivi d'un deuxième signal d'entraînement (502) présentant sensiblement l'amplitude du premier signal d'entraînement (501) ;
**caractérisé en ce que**
le deuxième signal d'entraînement (502) présente un signe opposé au signe du premier signal d'entraînement (501), le premier signal d'entraînement (501) et le deuxième signal d'entraînement (502) fournissent une onde carrée, et les amplitudes des premier et deuxième signaux d'entraînement (501, 502) comprennent une amplitude maximale.

13. Support tangible, non transitoire et lisible par ordinateur tel que revendiqué dans la revendication 12, dans lequel les instructions amènent en outre le dispositif de commande (120) à appliquer de manière répétée les premier et deuxième signaux d'entraînement (501, 502) jusqu'à ce qu'un transducteur atteigne un fonctionnement dynamique.

14. Le support tangible, non transitoire et lisible par ordinateur selon la revendication 13, dans lequel lorsque le transducteur atteint un fonctionnement dynamique, les instructions amènent le dispositif de commande (120) à fonctionner dans un mode de fonctionnement en boucle fermée.

15. Support tangible, non transitoire et lisible par ordinateur selon la revendication 12, dans lequel l'initialisation du dispositif d'imagerie ultrasonore (110) surmonte la friction de composants mécaniques du dispositif d'imagerie ultrasonore (110).
